Europäisches Patentamt

European Patent Office

Office européen des brevets

(11) Veröffentlichungsnummer: **0 422 331 B1**

(12) **EUROPÄISCHE PATENTSCHRIFT**

(45) Veröffentlichungstag der Patentschrift: **06.04.94**

(21) Anmeldenummer: **90108634.8**

(22) Anmeldetag: **08.05.90**

(51) Int. Cl.⁵: **F21M 1/00**

(54) **Operationsleuchte mit verstellbarer Halterung.**

(30) Priorität: **07.10.89 DE 3933596**

(43) Veröffentlichungstag der Anmeldung:
**17.04.91 Patentblatt 91/16**

(45) Bekanntmachung des Hinweises auf die
Patenterteilung:
**06.04.94 Patentblatt 94/14**

(84) Benannte Vertragsstaaten:
**DE FR GB IT**

(56) Entgegenhaltungen:
**EP-A- 0 299 196**
**EP-A- 0 330 382**

(73) Patentinhaber: **Heraeus Instruments GmbH**
**Heraeusstrasse 12-14**
**D-63450 Hanau(DE)**

(72) Erfinder: **Kloos, Thomas**
**Friedensstrasse 15**
**D-6050 Offenbach am Main(DE)**

(74) Vertreter: **Kühn, Hans-Christian**
**Heraeus Holding GmbH,**
**Stabsstelle Schutzrechte,**
**Heraeusstrasse 12-14**
**D-63450 Hanau (DE)**

# Beschreibung

Die Erfindung betrifft ein Verfahren zum Betrieb einer Operationsleuchte mit wenigstens einem aus dem Boden ihres Leuchtengehäuses austretenden Lichtbündel, wobei die Bewegung der Leuchte mittels einer kardanisch verstellbaren Halterung, welche über wenigstens ein mit horizontaler Drehachse versehenes Kippgelenk mit einem als Deckenstativ ausgebildeten Drehgelenk mit vertikaler Drehachse verbunden ist, bewirkt wird, wobei die Leuchtenachse durch mindestens ein Stellglied auf ein Operationsfeld als Beleuchtungsbereich ausgerichtet wird und wobei das Stellglied zwecks automatischer Nachlaufregelung von einem mit akustischen oder elektromagnetischen Signalen arbeitenden Entfernungsmesser angesteuert wird, sowie eine Operationsleuchte.

Aus der DE-OS 37 23 009 ist eine Operationsleuchte mit mehreren aus dem Boden des Leuchtengehäuses austretenden Lichtbündeln bekannt, die so ausgerichtet werden, daß sie sich im Operationsfeld unter Bildung eines verstärkten Beleuchtungsfeldes überlagern. Die Ausrichtung erfolgt mit Hilfe eines in der Leuchte befindlichen Ultraschallentfernungsmessers, der ein den Abstand zwischen Leuchte und Operationsfeld entsprechendes Signal erzeugt und dieses als Führungsgröße einem Regelkreis zuführt; im Regelkreis wird diese Führungsgröße mit der als Regelgröße dienenden Winkelstellung der Lichtbündel verglichen und bei Regelabweichung so lange ein Stellsignal erzeugt, bis die Regelgröße der Führungsgröße entspricht. Durch die zugehörige elektrische Schaltung wird verhindert, daß im Strahlengang der Leuchte plötzlich auftauchende Meßobjekte, wie z.B. Hände oder Kopf des Chirurgen, die Lichtbündelung auf sich lenken. Die Freigabe zur Verstellung der Lichtbündel erfolgt daher entweder durch manuelle Betätigung eines am Leuchtengehäuse befindlichen Handgriffs oder durch Abstandsvergrößerung, wie sie sich beispielsweise bei einer zunehmenden Vertiefung der Operationswunde ergeben kann. Auf diese Weise ist ohne besondere Hilfsmittel eine automatische, störungsfreie Lichtbündelung zu erzielen.

Weiterhin ist aus der DE-OS 32 27 494 eine Operationsleuchte für zahn- und kiefertechnische Behandlungen bekannt, bei der ein Lichtbündel bei Bewegung des Behandlungstuhles durch automatische Nachführung der Lampe ständig auf den Mundbereich des Patienten ausgerichtet bleibt. Die zugehörige Nachführeinrichtung weist einen im Kopfbereich des Patienten befindlichen Ultraschallsender und im Behandlungsraum mehrere räumlich getrennte Ultraschallempfänger sowie eine Peilschaltung auf. Die Nachführung erfolgt über Servo- oder Schrittmotoren, durch die die Halterung der Lampe in bestimmte Posititonen bzw. Neigungen gebracht werden kann. Eine solche Operationsleuchte ist aufgrund der erforderlichen Unterbringung des Ultraschallsenders in unmittelbarer Nähe des zu beleuchtenden Operationsfeldes in der allgemeinen Chirurgie unbrauchbar. Hier sind insbesondere Einstellfehler sowie Schwierigkeiten bei Handhabung und Sterilisation zu erwarten.

Die Erfindung stellt sich die Aufgabe, bei beabsichtigter oder unbeabsichtigter Verstellung der Operationsleuchte die eingestellte Strahlungsrichtung so nachzustellen, daß der ursprünglich eingestellte Beleuchtungsbereich (Operationswunde) ohne Neueinstellung der Leuchte wieder exakt beleuchtet wird, wobei auch eine optimale Einstellung des Lichtbündels bzw. der Lichtbündel automatisch erfolgen soll.

Die Aufgabe wird erfindungsgemäß dadurch gelöst, daß die Raum-Koordinaten des Leuchtengehäuses und die Richtung seiner Leuchtenachse durch Winkelmessung an allen Gelenken und daß die Raum-Koordinaten des Beleuchtungsbereichs durch eine zusätzliche Entfernungsmessung entlang der Leuchtenachse ermittelt und durch Betätigung eines Bedienungselements als Koordinatenwerte gespeichert werden, daß nach einer Verstellung des Leuchtengehäuses dessen Position und die Ausrichtung seiner Leuchtenachse durch erneute Winkelmessung ermittelt und in Raum-Koordinaten dargestellt wird, daß aus den Differenzen zwischen den nach Verstellung des Leuchtengehäuses ermittelten und den vor dieser Verstellung gespeicherten Koordinatenwerten Stellsignale für mit den Gelenken der kardanischen Halterung verbundene Stellmotoren gebildet werden, und daß mittels der Stellmotoren das Leuchtengehäuses so lange verstellt wird, bis diese Leuchtenachse auf den ursprünglich eingestellten Beleuchtungsbereich auftrifft.

Bei einer Operationsleuchte mit nur einem einzigen Lichtbündel wird das Profil des Lichtbündels entlang der Gehäuseachse in Abhängigkeit von der Entfernung zwischen Leuchtengehäuse und Beleuchtungsbereich (Operationsfeld) durch Fokussierung optimal eingestellt und als digitaler Wert gespeichert.

Bei einer Operationsleuchte mit mehreren Lichtbündeln, die durch wenigstens ein Stellglied auf das Beleuchtungsfeld so auszurichten sind, daß sie sich unter Bildung eines verstärkten Beleuchtungsbereiches überlagern, werden nach Einstellung des verstärkten Beleuchtungsbereiches (Operationsfeld) dessen Koordinaten sowie die Koordinaten des Leuchtengehäuses durch Winkelmessung an den Gelenken und durch Entfernungsmessung zwischen verstärktem Beleuchtungsbereich und Lampengehäuse ermittelt und als digitale Werte gespeichert Nach Verstellung werden die Lichtbündel in Abhängigkeit von der Entfernung zwischen Leuchtengehäuse und Beleuchtungsbereich so eingestellt, daß sie sich unter Bildung eines verstärkten Beleuchtungsbereiches wiederum überlagern.

Die von den Winkelmessern und dem Entfernungsmesser ermittelten Werte werden durch Koordinaten-Tranformation in kartesische Raum-Koordinaten umgewandelt, wobei aus den Differenzen der ursprünglichen Koordinatenwerte und der nach Verstellung ermittelten Koordinatenwerte auf den Achsen des X-, Y-, Z-Koordinatensystems Stellsignale für die Stellmotoren abgeleitet werden. Die Raum-Koordinaten des Beleuchtungsfeldes (Operationsfeldes) bilden dabei den Sollwert.

In einer erfindungsgemäßen Vorrichtung zur Durchführung des Verfahrens sind wenigstens die beiden Gelenke der kardanisch verstellbaren Halterung mit Stellmotoren versehen, wobei alle Gelenke der Halterung mit Winkelgebern versehen sind. Winkelgeber und Entfernungsmesser sind an den Eingang eines elektronischen Rechners angeschlossen, welcher die von den Winkelgebern und dem Entfernungsmesser abgegebenen Werte speichert und über wenigstens zwei Ausgänge mit jeweils einem Stellmotor zur Verstellung der kardanischen Halterung des Leuchtengehäuses verbunden ist. Die Einstellung des optimalen Beleuchtungsbereiches erfolgt durch einen mit dem Leuchtengehäuse verbundenen Handgriff, welcher zu Sterilisationszwecken abnehmbar ist. Der Handgriff ist mit einer Drucktaste versehen, welche bei optimaler Einstellung durch Tastenbetätigung die Koordinatenwerte speichert. Es ist jedoch auch möglich, einen Handgriff vorzusehen, welcher nach Loslassen des Bedienungspersonals die Koordinatenwerte speichert, wobei die Leuchte anschließend über seitliche Griffelemente bewegt wird.

Da die Halterungselemente, d.h. die mit den Gelenken verbundenen Stäbe oder Bügel, unveränderliche Dimensionen haben, ist es möglich, die Position und Ausrichtung des Leuchtengehäuses nur durch Messung der Winkel an den Gelenken bzw. in den Gelenken zu ermitteln. Die Entfernung zwischen Leuchte und Operationsfeld erfolgt durch Entfernungsmessung auf akustischer oder elektromagnetischer Basis. Durch Koordinatentransformation werden aus den gemessenen Winkeln und der Entfernung zum Operationsfeld die Raum-Koordinaten von Leuchtengehäuse und Operationsfeld errechnet und abgespeichert. Die nach Verstellung der Leuchte ermittelten Abweichungen im Koordinatensystem dienen als Stellsignale für die in der kardanischen Halterung des Leuchtengehäuses befindlichen Stellmotoren.

Die mit der Erfindung erzielbaren Vorteile sind insbesondere darin zu sehen, daß Winkelgeber, Stellmotoren, Rechner und Bedienungselemente sich ohne Probleme in bisher übliche Operationsleuchten integrieren lassen, so daß auf den Aufbau zusätzlicher Apparaturen oder Gehäuse verzichtet werden kann. Ein weiterer Vorteil ist darin zu sehen, daß die Winkelgeber im vollen 360°-Kreis arbeiten können, so daß stets eine eindeutige Zuordnung der Position und Ausrichtung des Leuchtengehäuses möglich ist. Darüber hinaus dient die Entfernungsmessung auch zur Überprüfung er ermittelten Koordinatenwerte.

Im folgenden ist der Gegenstand anhand der Figuren 1 bis 4 näher erläutert. Figur 1 zeigt in einer perspektivischen Darstellung die Halterung zusammen mit dem nur schematisch dargestellten Leuchtengehäuse. Figur 2 zeigt eine Operationsleuchte mit einer einzigen Lichtquelle, wobei das Leuchtengehäuse im Querschnitt dargestellt ist. Figur 3 zeigt eine Seitenansicht einer mehrstrahligen Operationsleuchte, während in Figur 4 die geometrischen Beziehungen nach Verstellung der Operationsleuchte dargestellt sind. Die mit $\phi$ (Phi) bezeichneten Winkel sind jeweils mit dem Index des zugehörigen Gelenks bzw. der zugehörigen Koordinatenachse versehen, wobei die Winkel zwecks besserer Übersicht nur zum Teil dargestellt sind.

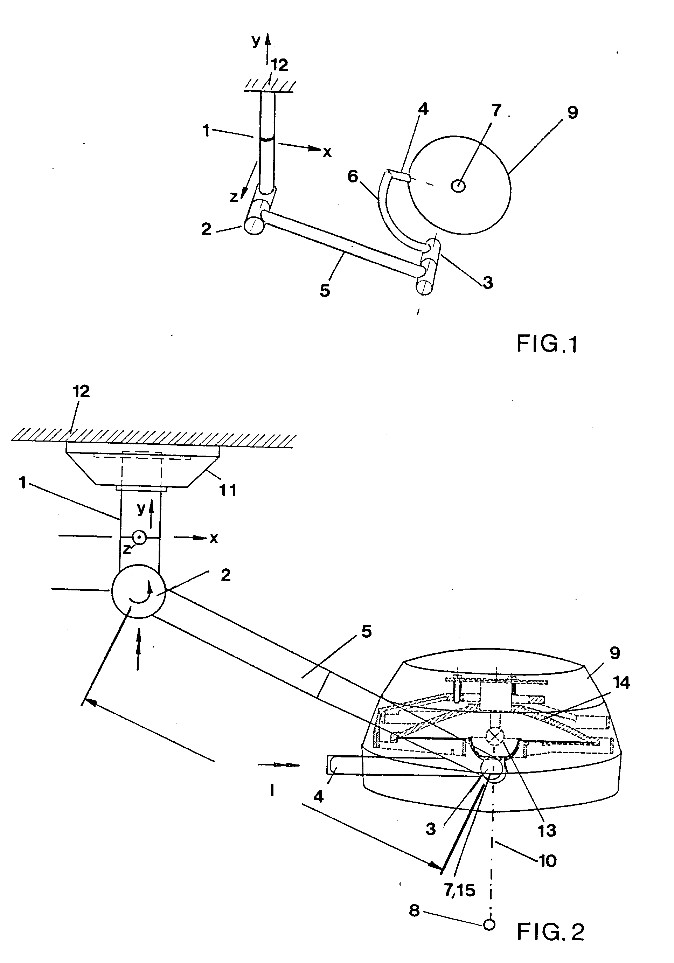

Gemäß Figur 1 weist die als Deckenstativ ausgebildete Halterung der Operationsleuchte ein an der Decke 12 befestigtes Drehgelenk 1 auf, dessen unterer Teil um eine vertikale Achse um 360° drehbar ist. Im Unterteil des Drehgelenks 1 ist ein um eine horizontale Achse verstellbares Kippgelenk 2 angeordnet, dessen Verstellbereich etwa 180° beträgt. Das Kippgelenk 2 ist über einen Stab 5 mit dem Drehgelenk 3 verbunden, welches eine horizontale Drehachse aufweist und um 360° drehbar ist. Drehgelenk 3 ist mit einem Bügel 6 verbunden, welcher an seinem Ende ein weiteres Drehgelenk 4 aufweist, dessen Achse senkrecht zu der des Drehgelenks 3 verläuft. Drehgelenk 4 ist mit dem eigentlichen Leuchtengehäuse 9 der Operationsleuchte verbunden. Die beiden Drehgelenke 3 und 4 bilden die kardanische Halterung der Operationsleuchte und damit die eigentlichen Drehachsen des Leuchtengehäuses, wobei sich ihre Drehachsen im Referenzpunkt 7 treffen.

Die den Gelenkbezifferungen entsprechenden Winkel $\phi_1$, $\phi_2$, $\phi_3$ und $\phi_4$ werden über in den Gelenken befindliche Winkelmesser ermittelt und an einen Rechner zwecks Koordinatenermittlung weitergeleitet. Als Winkelmesser werden in den Gelenken 1, 2, 3 und 4 optisch abtastbare Kodierwalzen verwendet, die ein dem Drehwinkel des Gelenkes entsprechendes digitales Signal erzeugen. Die Winkelmesser sind zwecks besserer Übersicht nicht dargestellt, ebenso wie die zur Verstellung des Leuchtengehäuses in den Gelenken 3 und 4 erforderlichen Stellmotoren. Bei den Stellmotoren ist jeweils das zu verstellende Teil mit dem Rotor verbunden, d. h. daß im Gelenk 3 der Rotor des Stellmotors mit dem Bügel 6, im Gelenk 4 der Rotor mit dem Leuchtengehäuse 9 verbunden ist. Dabei ist es selbstverständlich auch möglich, die Drehung jeweils mit Hilfe eines Getriebes zu übertragen.

Gemäß Figur 2 ist das Drehgelenk 1 mittels Konsole 11 an der Decke 12 befestigt. Die Bezeichnung der Gelenke entspricht dabei der anhand von Figur 1 erläuterten Ausführungsform. Das im Querschnitt dargestellte Leuchtengehäuse 9 weist eine einzige Lichtquelle 13 auf, deren abgestrahltes Licht mit Hilfe einer Reflektoranordnung 14 (Kaltlichtreflektor) in Richtung Leuchtenachse 10 gebündelt wird. Eine Fokussierung ist durch Abstandsveränderung zwischen Lichtquelle 13 und Reflektor 14 möglich. Im Bereich des Referenzpunktes 7 ist ein Ultraschallsensor 15 zur Ermittlung der Entfernung zwischen Beleuchtungsbereich 8 (Operationsebene) und dem Referenzpunkt 7 des Leuchtengehäuses 9 angeordnet.

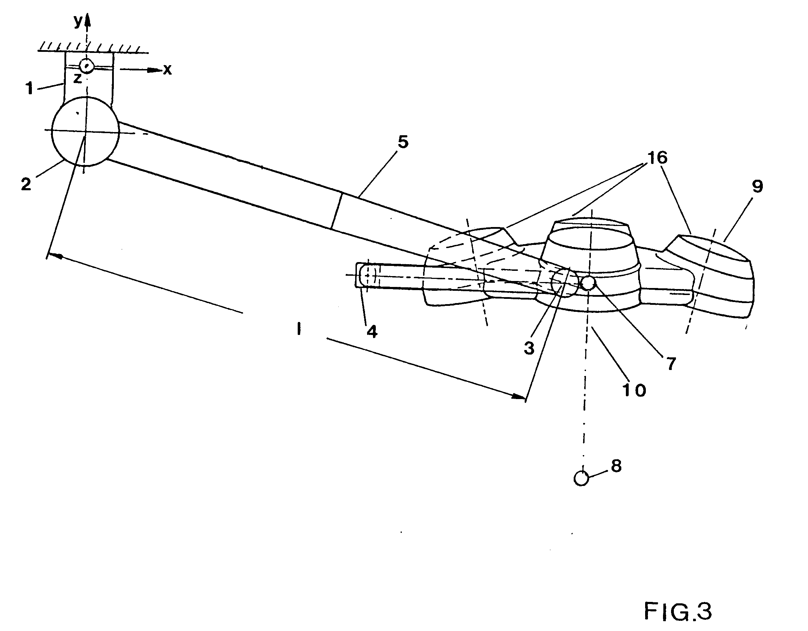

Figur 3 zeigt eine Operationsleuchte mit einem mehrstrahligen Leuchtengehäuse, das aus einzelnen Strahlern 16 besteht, die ringartig auf einem Träger angeordnet sind. Das aus den Strahlern austretende Licht wird mit Hilfe eines Ultraschallsensors unter Bildung eines verstärkten Beleuchtungsbereiches in der Operationsebene 8 gebündelt. Der Ultraschallsensor bildet ein von der Entfernung abhängiges Signal, das einem Regelkreis zugeführt wird, der die Achsen der Lichtbündel der einzelnen Strahler mittels Stellglied so ausrichtet, daß der verstärkte Beleuchtungsbereich trotz Abstandsänderung erhalten bleibt.

Die Position der Gelenke wird gemäß Figur 3 folgendermaßen ermittelt: Im Gelenk 1 befindet sich der Ursprung des kartesischen Koordinatensystems, wobei sich die Richtung x in horizontaler Richtung erstreckt, während die Richtung y entlang der Drehachse des Drehgelenks 1 verläuft. Die Richtung z steht senkrecht auf der durch x und y ausgespannten Ebene und tritt nach oben aus der Zeichenebene aus. Die Position des Gelenks 3 ist dabei abhängig von der Länge des Stabes 5 und der Drehungen um die Gelenke 1 und 2, welche mit den Winkeln $\phi_1$ und $\phi_2$ bezeichnet werden. Die Länge des Stabes 5 wird mit 1 bezeichnet:

$$x_3 = l \cdot \cos\phi_2 \cdot \cos\phi_1$$
$$y_3 = l \cdot \sin\phi_2$$
$$z_3 = l \cdot \cos\phi_2 \cdot \sin\phi_1$$

Für die Koordinaten des Punktes 7 ergeben sich folgende Beziehungen, wobei mit $y_2$ der Abstand der Drehachse des Gelenks 2 am Nullpunkt bezeichnet ist; der Abstand zwischen dem Drehgelenk 3 und Punkt 7 wird mit m bezeichnet:

$$x_7 = o + l \cdot \cos\phi_2 \cdot \cos\phi_1 + o$$
$$y_7 = y_2 + l \cdot \sin\phi_2$$
$$z_7 = o + l \cdot \cos\phi_2 \cdot \sin\phi_1 + m$$

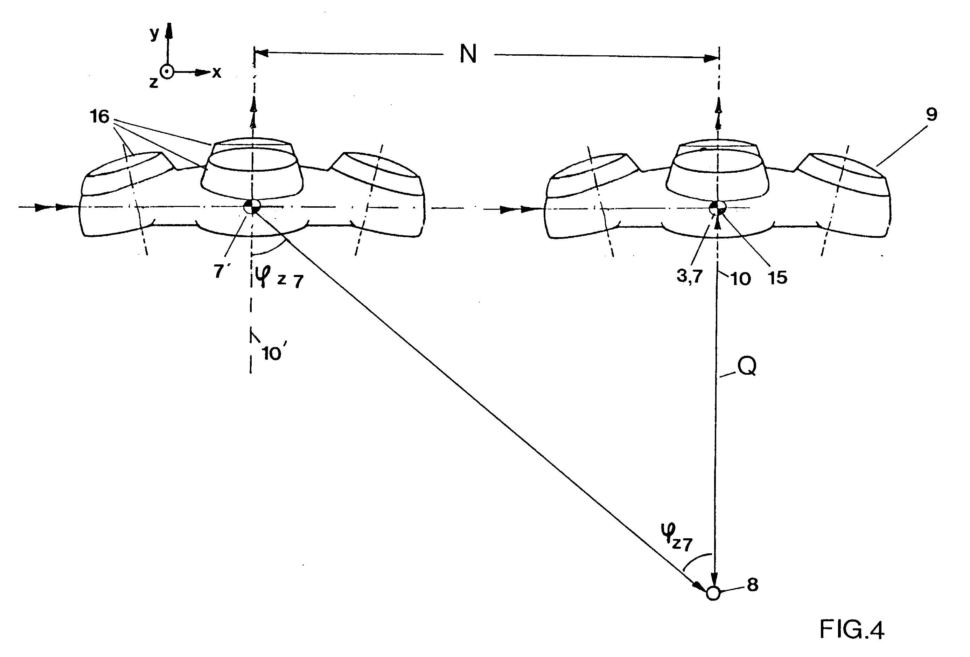

Gemäß Figur 4 ergibt sich für die Winkelstellung der Normalen auf dem Leuchtenkörper bezüglich des Koordinatensystems folgende Beziehung:

$$\phi z_7 = \phi_2 - \phi_3$$
$$\phi x_7 = \phi_4$$

Der die Operationsebene symbolisierende Punkt 8 wird mittels Entfernungsmessung bestimmt. Im einfachsten Fall steht die Leuchte, wie in Figur 4 dargestellt, mit ihrem Mittelpunkt 7 über der durch Ziffer 8 symbolisierten Operationsebene, wobei der Abstand zwischen Mittelpunkt 7 und Operationsebene 8 mit Q bezeichnet ist. Somit kann bei Auslenkung des Leuchtengehäuses 9 um die Länge N von Position 7 in Position 7' folgende Beziehung aufgestellt werden:

$$N_x = x_7 - x_{7'}$$
$$N_y = y_7 - y_{7'}$$
$$N_z = z_7 - z_{7'}$$

Gemäß Figur 4 gilt nun für die neue Winkelstellung folgende Beziehung:

$$\phi_{z_{7'}} = \arctan \frac{Q - N_y}{N_x}$$

$$\phi_{x_{7'}} = \arctan \frac{Q - N_y}{N_z}$$

Die so errechneten Winkel werden dann von den Stellmotoren durch Drehung um Gelenk 3 und Gelenk 4 eingestellt. Die Erstellung des verstärkten Beleuchtungsbereiches durch Überlagerung der Lichtbündel erfolgt, wie bereits eingangs angegeben, durch automatische Nachlaufregelung mittels Entfernungsmesser.

Es ist selbstverständlich möglich, zur Erweiterung der Verstellbarkeit der erfindungsgemäßen Operationsleuchte weitere Gelenke einzusetzen, die ebenfalls mit Winkelmessern ausgestattet sind.

**Patentansprüche**

1. Verfahren zum Betrieb einer Operationsleuchte mit wenigstens einem aus dem Boden ihres Leuchtengehäuses (9) austretenden Lichtbündel, wobei die Bewegung der Leuchte mittels einer kardanisch verstellbaren Halterung (3, 4), welche über wenigstens ein mit horizontaler Drehachse versehenes Kippgelenk (2) mit einem als Deckenstativ ausgebildeten Drehgelenk (1) mit vertikaler Drehachse verbunden ist, bewirkt wird, wobei die Leuchtenachse durch mindestens ein Stellglied auf ein Operationsfeld als Beleuchtungsbereich ausgerichtet wird und wobei das Stellglied zwecks automatischer Nachlaufregelung von einem mit akustischen oder elektromagnetischen Signalen arbeitenden Entfernungsmesser (15) angesteuert wird, dadurch gekennzeichnet, daß die Raum-Koordinaten des Leuchtengehäuses (9) und die Richtung seiner Leuchtenachse (10) durch Winkelmessung an allen Gelenken (1, 2, 3, 4) und daß die Raum-Koordinaten des Beleuchtungsbereichs durch eine zusätzliche Entfernungsmessung entlang der Leuchtenachse (10) ermittelt und durch Betätigung eines Bedienungselements als Koordinatenwerte gespeichert werden, daß nach einer Verstellung des Leuchtengehäuses (9) dessen Position und die Ausrichtung seiner Leuchtenachse (10) durch erneute Winkelmessung ermittelt und in Raum-Koordinaten dargestellt wird, daß aus den Differenzen zwischen den nach Verstellung des Leuchtengehäuses ermittelten und den vor dieser Verstellung gespeicherten Koordinatenwerten Stellsignale für mit den Gelenken (3, 4) der kardanischen Halterung verbundene Stellmotoren gebildet werden, und daß mittels der Stellmotoren das Leuchtengehäuse (9) so lange verstellt wird, bis diese Leuchtenachse (10) auf den ursprünglich eingestellten Beleuchtungsbereich (8) auftrifft.

2. Verfahren nach Anspruch 1, dadurch gekennzeichnet, daß das Profil des bzw. der Lichtbündel entlang der Leuchtenachse (10) in Abhängigkeit von der Entfernung zwischen Leuchtengehäuse (9) und Beleuchtungsbereich (8) eingestellt wird.

3. Verfahren nach Anspruch 1 oder 2 zum Betrieb einer Operationsleuchte mit wenigstens zwei aus dem Boden ihres Leuchtengehäuses austretenden Lichtbündeln, die sich unter Bildung eines verstärkten Beleuchtungsbereiches überlagern, dadurch gekennzeichnet, daß nach Einstellung des verstärkten Beleuchtungsbereiches (8) dessen Koordinaten sowie die Koordinaten des Leuchtengehäuses (9) und dessen Ausrichtung durch Winkelmessung an den Gelenken (1, 2, 3, 4) und durch Entfernungsmessung zwischen verstärktem Beleuchtungsbereich (8) und Leuchtengehäuse (9) ermittelt und als Koordinaten-Werte gespeichert und die Lichtbündel in Abhängigkeit von der Entfernung zwischen Leuchtengehäuse und Beleuchtungsbereich so eingestellt werden, daß sie sich unter Bildung eines verstärkten Beleuchtungsbereiches überlagern.

4. Verfahren nach einem der Ansprüche 1 bis 3, dadurch gekennzeichnet, daß die von den Winkelmessern und dem Entfernungsmesser (15) ermittelten Werte in kartesische Raum-Koordinaten umgewandelt und aus den Differenzen der jeweiligen Werte auf den Achsen des X-, Y-, Z-Koordinatensystems Stellsignale für die Stellmotoren gebildet werden.

5. Operationsleuchte mit wenigstens einem aus dem Boden ihres Leuchtengehäuses (9) austretenden Lichtbündel, wobei das Leuchtengehäuse (9) in einer kardanischen Halterung (3, 4) angeordnet ist und über wenigstens ein mit horizontaler Drehachse versehenes Kippgelenk (2) mit wenigstens einem weiteren Drehgelenk (1) mit vertikaler Drehachse verbunden ist, wobei die Leuchtenachse (10) durch mindestens ein Stellglied auf ein Operationsfeld als Beleuchtungsbereich ausrichtbar ist, wobei das Stellglied zwecks automatischer Nachlaufregelung von einem mit akustischen oder elektromagnetischen Signalen arbeitenden Entfernungsmesser (15) verbunden ist, dadurch gekennzeichnet, daß wenigstens die beiden Gelenke (3, 4) der kardanischen Halterung mit Stellmotoren versehen sind, daß alle Gelenke (1, 2, 3, 4) der Halterung mit Winkelgebern versehen sind, daß die Winkelgeber und die Entfernungsmesser (15) an den Eingang eines elektronischen Rechners angeschlossen sind, welcher die von den Winkelgebern und dem Entfernungsmesser (15) abgegebenen Werte speichert und daß der Rechner wenigstens zwei Ausgänge aufweist, welche jeweils mit einem Stellmotor zur Verstellung der kardanischen Halterung des Leuchtengehäuses (9) elektrisch verbunden sind.

6. Operationsleuchte nach Anspruch 5, dadurch gekennzeichnet, daß wenigstens zwei Lichtbündel aus dem Boden des Leuchtengehäuses (9) austreten, deren optische Achsen durch mindestens ein Stellglied auf ein Operationsfeld so ausrichtbar sind, und daß sich die Lichtbündel unter Bildung eines verstärkten Beleuchtungsbereichs (8) überlagern.

7. Operationsleuchte nach Anspruch 5 oder 6, dadurch gekennzeichnet, daß die Winkelgeber wenigstens teilweise mit optisch abtastbaren Kodierscheiben oder Kodierwalzen versehen sind.

**8.** Operationsleuchte nach Anspruch 5 oder 6, dadurch gekennzeichnet, daß die Winkelgeber wenigstens teilweise mit Hall-Generatoren ausgestattet sind.

**Claims**

**1.** A method for the operation of a surgical operation light with at least one light beam emerging from the base of its light housing (9), in which the movement of the light is brought about by means of a gimbally adjustable mounting (3,4), which is connected via at least one tilting joint (2), provided with a horizontal rotation axis, with a swivel joint (1), constructed as a ceiling support, with a vertical rotation axis, in which the light axis is aligned by at least one adjustment member onto an operation field as lighting area and in which the adjustment member, for the purpose of automatic follow-up control, is controlled by a distance meter (15) operating with acoustic or electromagnetic signals, characterised in that the space coordinates of the light housing (9) and the direction of its light axis (10) are determined by angle measurement on all joints (1,2,3,4) and that the space coordinates of the lighting area are determined by an additional distance measurement along the light axis (10) and are stored as coordinate values by actuation of an operating element, that after an adjustment of the light housing (9),its position and the alignment of its light axis (10) is determined by further angle measurement and is represented in space coordinates, that from the differences between the coordinate values determined after adjustment of the light housing and those stored before this adjustment, adjustment signals are formed for adjusting motors connected with the joints (3,4) of the gimbal mounting, and that the light housing (9) is adjusted by means of the adjusting motors until this light axis (10) meets the originally set lighting area (8).

**2.** A method according to Claim 1, characterised in that the section of the light beam or beams along the light axis (10) is set as a function of the distance between the light housing (9) and the lighting area (8).

**3.** A method according to Claim 1 or 2 for the operation of a surgical operation light with at least two light beams emerging from the base of its light housing, which beams overlie each other, forming an intensified lighting area, characterised in that after the setting of the intensified lighting area (8), its coordinates and also the coordinates of the light housing (9) and its alignment are determined by angle measurement on the joints (1,2,3,4) and through distance measurement between intensified lighting area (8) and light housing (9), and are stored as coordinate values and the light beams are set as a function of the distance between the light housing and the lighting area such that they overlie each other, forming an intensified lighting area.

**4.** A method according to one of Claims 1 to 3, characterised in that the values determined by the angle meters and the distance meter (15) are converted into cartesian space coordinates and adjustment signals for the adjusting motors are formed from the differences of the respective values on the axes of the X-, Y-, Z-coordinate system.

**5.** An operation light with at least one light beam, emerging from the base of its light housing (9), in which the light housing (9) is arranged in a gimbal mounting (3,4) and is connected via at least one tilting joint (2) provided with a horizontal rotation axis with at least one further swivel joint (1) with a vertical rotation axis, in which the light axis (10) is able to be aligned by at least one adjustment member onto an operation field as lighting area, in which the adjustment member, for the purpose of automatic follow-up control, is connected with a distance meter (15) operating with acoustic or electromagnetic signals, characterised in that at least the two joints (3,4) of the gimbal mounting are provided with adjusting motors, that all the joints (1,2,3,4) of the mounting are provided with angle indicators, that the angle indicators and the distance meters (15) are connected to the input of an electronic computer, which stores the values delivered from the angle indicators and from the distance meter (15) and that the computer has at least two outputs, which in each case are connected electrically with an adjusting motor for the adjustment of the gimbal mounting of the light housing (9).

**6.** An operation light according to Claim 5, characterised in that at least two light beams emerge from the base of the light housing (9), the optical axes of which are able to be aligned onto an operation field by at least one adjustment member, and that the light beams overlie each other, forming an intensified lighting area (8).

7. An operation light according to Claim 5 or 6, characterised in that the angle indicators are provided at least partially with code discs or code rolls which are able to be scanned optically.

8. An operation light according to Claim 5 or 6, characterised in that the angle indicators are at least partially equipped with Hall generators.

**Revendications**

1. Procédé de fonctionnement d'une lampe de salle d'opérations comportant au moins un faisceau lumineux émergeant du fond de son boîtier de lampe (9), le mouvement de la lampe étant actionné par un support (3, 4) réglable à la Cardan, qui est relié par au moins une articulation basculante (2) pourvue d'un axe de rotation horizontal, à une articulation à charnière (1) à axe de rotation vertical, réalisée comme un support de plafond, l'axe de la lampe étant orienté par au moins un organe de positionnement sur un champ opératoire qui constitue la zone d'éclairage et l'organe de positionnement, dans le but d'un réglage automatique de position, étant commandé par un télémètre (15) fonctionnant avec des signaux acoustiques ou électromagnétiques, caractérisé en ce que les coordonnées spatiales du boîtier de lampe (9) et la direction de son axe de lampe (10) sont calculées par mesure angulaire sur toutes les articulations (1, 2, 3, 4) et en ce que les coordonnées spatiales de la zone d'éclairage sont calculées par télémétrie supplémentaire le long de l'axe d'éclairage (10) et mémorisées en tant que valeurs de coordonnées en actionnant un élément de commande, en ce qu'après un réglage du boîtier de lampe (9), sa position et l'orientation de son axe de lampe (10) sont calculées par une nouvelle mesure angulaire et représentées sous la forme de coordonnées spatiales, en ce qu'à partir des différences entre les valeurs de coordonnées calculées après le réglage du boîtier et les valeurs de coordonnées mémorisées avant ce réglage on forme des signaux de réglage pour des moteurs de réglage reliés aux articulations (3, 4) du support à la Cardan, et en ce qu'au moyen des moteurs de réglage, le boîtier d'éclairage (9) est réglé aussi longtemps que cet axe de lampe (10) apparaît sur la zone d'éclairage (8) réglée initialement.

2. Procédé selon la revendication 1, caractérisé en ce que le profil de ou des faisceaux lumineux le long de l'axe de lampe (10) est réglé en fonction de la distance entre le boîtier de lampe (9) et la zone d'éclairage (8).

3. Procédé selon la revendication 1 ou 2, de fonctionnement d'une lampe de salle d'opérations, comportant au moins deux faisceaux lumineux émergeant du fond de leur boîtier de lampe, qui se chevauchent en formant une zone d'éclairage renforcée, caractérisée en ce qu'après le réglage de la zone d'éclairage (8) renforcée, ses coordonnées ainsi que les coordonnées du boîtier de lampe (9) et son orientation sont calculées par mesure angulaire sur les articulations (1, 2, 3, 4) et par la mesure de la distance entre la zone d'éclairage (8) renforcée et le boîtier de lampe (9) et mémorisées comme valeurs de coordonnées et en ce que les faisceaux lumineux sont réglés en fonction de la distance entre le boîtier de lampe et la zone d'éclairage, de telle manière qu'ils se chevauchent en formant une zone d'éclairage renforcée.

4. Procédé selon l'une des revendications 1 à 3, caractérisé en ce que les valeurs calculées par les goniomètres et le télémètre (15) sont transformées en coordonnées spatiales cartésiennes et en ce qu'à partir des différences des valeurs respectives sur les axes du système de coordonnées x, y, z, on forme des signaux de réglage pour les moteurs de réglage.

5. Lampe pour salle d'opérations comportant au moins un faisceau émergeant du fond de son boîtier de lampe (9), le boîtier de lampe (9) étant agencé dans un support (3, 4) à la Cardan et est relié par au moins une articulation basculante (2) pourvue d'un axe de rotation horizontal, à au moins une autre articulation à charnière (1) à axe de rotation vertical, l'axe de la lampe (10) étant orienté par au moins un organe de positionnement sur un champ opératoire qui constitue la zone d'éclairage et l'organe de positionnement, dans le but d'un réglage automatique de position, étant commandé par un télémètre (15) fonctionnant avec des signaux acoustiques ou électromagnétiques, caractérisé en ce qu'au moins les deux articulations (3, 4) du support à la Cardan sont pourvues de moteurs de réglage, en ce que toutes les articulations (1, 2, 3, 4) du support sont pourvus de capteurs angulaires, en ce que les capteurs angulaires et le télémètre (15) sont branchés à l'entrée d'un ordinateur qui mémorise les valeurs fournies par les capteurs angulaires et le télémètre (15) et en ce que l'ordinateur présente au moins deux sorties qui sont chacune reliées électriquement à un moteur de réglage pour régler le support à la Cardan du boîtier de lampe (9).

6. Lampe de salle d'opérations selon la revendication 5, caractérisée en ce qu'au moins deux faisceaux lumineux émergent du fond du boîtier de lampe (9), dont les axes optiques peuvent être orientés par au moins un organe de positionnement sur un champ d'opération de telle manière que les faisceaux lumineux se chevauchent pour former une zone d'éclairage (8) renforcée.

7. Lampe de salle d'opérations selon la revendication 5 ou 6, caractérisée en ce que les capteurs angulaires sont au moins partiellement pourvus de disques de codage ou de rouleaux de codage à balayage optique.

8. Lampe de salle d'opérations selon la revendication 5 ou 6, caractérisée en ce que les capteurs angulaires sont au moins partiellement équipés de générateurs Hall.

FIG.1

FIG.2

9

FIG.3

FIG.4

EP 0 422 331 B1